(19) Europäisches Patentamt
European Patent Office
Office européen des brevets

(11) Publication number : **0 160 218**
**B1**

(12) # EUROPEAN PATENT SPECIFICATION

(45) Date of publication of patent specification : **21.10.87**

(51) Int. Cl.[4] : **C 07 C143/78**

(21) Application number : **85103687.1**

(22) Date of filing : **28.03.85**

(54) **Process for preparing C8 -C24 alkylbenzene sulfonylazides.**

(30) Priority : **02.04.84 US 595779**

(43) Date of publication of application :
**06.11.85 Bulletin 85/45**

(45) Publication of the grant of the patent :
**21.10.87 Bulletin 87/43**

(84) Designated contracting states :
**CH DE FR GB IT LI NL**

(56) References cited :
**DE-A- 2 245 611**
**US-A- 4 284 575**

(73) Proprietor : **MERCK & CO. INC.**
**126, East Lincoln Avenue P.O. Box 2000**
**Rahway New Jersey 07065-0900 (US)**

(72) Inventor : **Roberts, Edward F.**
**220 Valley Road**
**Princeton New Jersey 08540 (US)**
Inventor : **Pines, Seemon H.**
**24 Candlewood Drive**
**Murray Hill New Jersey 07974 (US)**
Inventor : **Russ, Warren K., Jr.**
**209 Hillcrest Drive**
**Piscataway New Jersey 08854 (US)**

(74) Representative : **Abitz, Walter, Dr.-Ing. et al**
**Abitz, Morf, Gritschneder, Freiherr von Wittgenstein**
**Postfach 86 01 09**
**D-8000 München 86 (DE)**

**EP 0 160 218 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

Background of the invention

4-Dodecylbenzenesulfonylazides (p-azidosulfonyldodecylbenzenes) were disclosed in U.S. Patent 4,284,575 and have proven to be useful as diazo transfer reagents, such as in the following :

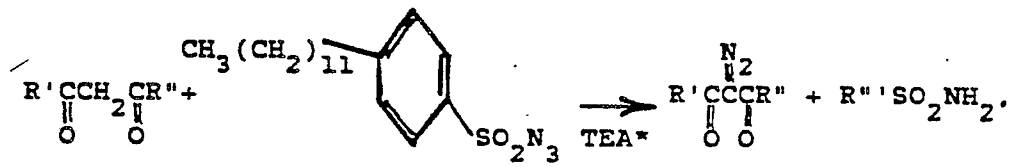


* (Triethylamine)

wherein R′ and R″ are, inter alia, alkyl, aryl, cycloalkyl, heterocyclic, aralkyl, lower alkoxy, and alkylthio ; and

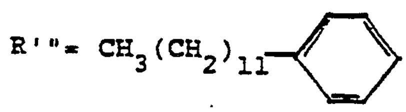

In reaction efficiency, 4-dodecylbenzenesulfonylazides compare favorably with conventionally known diazo transfer reagents, but offer the significant safety advantages of shock stability and nonexplosiveness (Hazen et al., Synth. Comm., 1981, 11, 947). In contrast, certain other azide diazo transfer reagents, including the widely-used p-tosylazide (p-toluenesulfonylazide), are shock sensitive and explosive. In fact, p-tosylazide has the shock sensitivity of tetryl and the explosive power of TNT, and two explosions have been reported during its preparation.

However, the process for preparing 4-dodecylbenzenesulfonylazides which was disclosed in U.S. Patent 4,284,575 includes a number of problems. The two-step process of that reference involves reacting dodecylbenzenesulfonic acids with phosphorus oxychloride at 105 °C, then isolating the sulfonyl chlorides as an oil and reacting them with sodium azide in acetone at 25 °C, in order to convert it to the azide. This process includes a troublesome, but necessary, quench after the first reaction step, plus an elaborate and time-consuming series of extractions and back-extractions of the dodecylbenzenesulfonylchlorides-in-ethyl acetate layer. Then, acetone must replace the ethyl acetate as the solvent for the second step, and an excess of solid sodium azide, which is insoluble in acetone, must be used for the conversion reaction. This use of excess sodium azide for the conversion reaction results in a sodium chloride cake, contaminated with sodium azide, which must be disposed of, and the resulting azide solution must be purified on silica gel, with the use of large volumes of solvents. Finally, the azide solution must be concentrated, which is always a potential hazard, despite its decreased sensitivity. For all these troubles, there is only a 72 % yield of 4-dodecylbenzenesulfonylazides by this process.

Consequently, it is an object of the present invention to develop an improved process for the synthesis of $C_8$-$C_{24}$ alkylbenzenesulfonylazides, particularly p-dodecylbenzenesulfonylazides, which is safe, convenient and offers a high yield.

Description of the invention

The instant invention is directed to a new, simple, and high-yielding process for the production of $C_8$-$C_{24}$ alkylbenzenesulfonylazides,

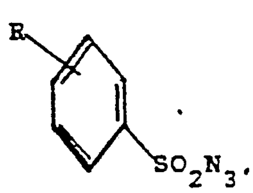

wherein R is a $C_8$-$C_{24}$ alkyl group, preferably dodecyl.

The process according to this invention comprises two steps. In the first step, $C_8$-$C_{24}$-alkylbenzenesulfonic acids, in a suitable nonreactive, water-immiscible solvent (such as a hydrocarbon or chlorinated solvent which is compatible with a reactive chlorinating agent, such as thionyl chloride, e. g., hexane, toluene, methylene chloride, and like solvents, preferably hexane), are reacted with an excess of

a reactive chlorinating agent, such as thionyl chloride, phosphoryl chloride, phosgene and other agents suitable for forming acid chlorides, particularly thionyl chloride, in the presence of a suitable solvent capable of forming a Vilsmeier reagent, such solvents including dimethylformamide, n-alkyl-pyrrolidinone or n-formyl-piperidine, preferably dimethylformamide, at 40° to 70 °C, preferably about 70 °C, to form the corresponding sulfonyl chlorides ($C_8$-$C_{24}$ alkylbenzenesulfonylchlorides). The hexane layer is then washed with sodium bicarbonate to remove any remaining $C_8$-$C_{24}$ alkylbenzenesulfonic acids. In the second step, the sulfonyl chlorides, still in the presence of the same solvent, are converted to the azides by reaction with aqueous sodium azide, in the presence of a anion transfer phase transfer catalyst (such as quaternary ammonium or phosphonium salts, including benzyltriethyl ammonium chloride, tetra-n-butyl ammonium chloride, cetyl-trimethyl ammonium chloride, or particularly Aliquat® 336 tri-n-alkylmethyl ammonium chloride — a mixture of $C_8$ and $C_{10}$ chains, with $C_8$ predominating — from General Mills Chemicals, Inc. ; macrocyclic or Macrobicyclic « crown » ethers ; triazine ethers ; or N-alkyl phosphoramides) :

$$R'''SO_3H + SOCl_2 \xrightarrow[\text{Hexane/ 70 °C.}]{\text{DMF}} R'''SO_2Cl \xrightarrow[\text{Aliquat 336}]{NaN_3,\ H_2O} R'''SO_2N_3,$$

wherein R''' =

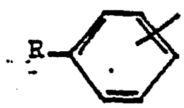

with R representing a $C_8$-$C_{24}$ alkyl group.

By the use of this new phase transfer catalysis method, troublesome solvent changes are avoided and the hexane solution of sulfonylazides may be isolated without hazardous concentration. Further, the use of a significant excess of sodium azide is eliminated, as the driving force for the bimolecular reaction is provided by the phase transfer reaction, and overall, the work-up is much simpler and reaction times are significantly reduced from those required under the previous methods.

The following example further illustrates the process of the instant invention, without limiting it.

Example 1

A. Preparation of 4-Dodecylbenzenesulfonylchlorides

A solution of 60 g (0.184 mole) of 4-dodecylbenzenesulfonic acids (a mixture of normal and branched chain isomers from K & K Division, ICN Pharmaceuticals, Inc. Life Sciences Group, 8.64 ml of dimethylformamide and 60 ml of hexane was prepared in a 250 ml 3-necked round-bottomed flask equipped with a mechanical overhead stirrer, thermometer, addition funnel and reflux condenser. The mixture was heated to 70 °C and 22.1 ml (36.24 g, 0.304 mole) of thionyl chloride (reagent grade, Fisher Scientific) was added at a rate to maintain controlled reflux (the addition time was about one hour).

The dark solution was heated another 2 hours at 70 °C and cooled to 40 °C (the progress of the reaction was monitored by thin layer chromatography). While still warm (about 40 °C), the mixture was transferred to a separatory funnel and the dark lower layer was separated from the hexane solution. The hexane layer was cooled to 25 °C and washed 60 ml of 5 % aqueous sodium bicarbonate solution, with this bicarbonate wash solution being back-extracted with 36 ml hexane, and the combined hexane layers being treated with 3 g Nuclear® SA carbon (Westvaco Co.) and stirred for 2 hours at 25 °C. The carbon was then removed by filtration and the cake washed with three portions (12 ml each) of hexane.

B. Preparation of 4-Dodecylbenzenesulfonylazides

The hexane solution from Step A was placed in a 500 ml 3-necked round-bottomed flask fitted with a mechanical overhead stirrer. A solution of 11.6 g (0.178 mole) of sodium azide (based on total solids from above sulfonylchlorides) dissolved in 100 ml water and 2.0 g Aliquat® 336 tri-n-$C_8$-$C_{10}$ alkylmethylammonium chloride phase transfer catalyst (Aldrich Chem. Co.) was charged into the flask. Intermittent cooling with a cold water bath was employed to keep the temperature below 35 °C, and while the reactants were being stirred, the reaction was monitored by thin layer chromatography. The reaction was complete after about 4 hours at 25 °C.

The two-phase mixture was transferred to a separatory funnel and the aqueous layer was removed. The hexane layer was washed with 100 ml of 5 % aqueous sodium bicarbonate and dried over 28 g of anhydrous sodium sulfate. The drying agent was removed by filtration and the cake was washed with 20 ml hexane.

The concentration and purity of the resulting 4-dodecylbenzenesulfonylazides were determined by evaporation of a small sample to an oil of constant weight and visible spectrophotometric assay for azide (assay for sulfonylazides was adapted from Sewinski et al., Anal. Chem., 1982, 54, 846-47). The yield (based on sulfonic acids) of 4-dodecylbenzenesulfonylazides was 94.7 %.

**Claims**

1. A process for the preparation of $C_8$-$C_{24}$ alkylbenzenesulfonylazides comprising :
a) reacting $C_8$-$C_{24}$ alkylbenzenesulfonic acids in a suitable water-immiscible, nonreactive solvent with an excess of a reactive chlorinating agent, in the presence of a solvent capable of forming a Vilsmeier reagent, at between 40° and 70 °C to form $C_8$-$C_{24}$ alkylbenzenesulfonylchlorides and removing any remaining acids with a sodium bicarbonate wash, then
b) reacting said $C_8$-$C_{24}$ alkylbenzenesulfonylchlorides in said water-immiscible, nonreactive solvent with aqueous sodium azide in the presence of a phase transfer catalyst useful for anion transfer.
2. A process according to Claim 1, wherein the $C_8$-$C_{24}$ alkylbenzenesulfonylazides are 4-dodecylbenzenesulfonylazides.
3. A process according to Claims 1 or 2, wherein the suitable water-immiscible, nonreactive solvent is selected from hexane, toluene and methylene chloride.
4. A process according to Claim 3, wherein the suitable water-immiscible, nonreactive solvent is hexane.
5. A process according to any one of Claims 1 to 4, wherein the $C_8$-$C_{24}$ alkylbenzenesulfonic acids are reacted with thionyl chloride, phosphoryl chloride or phosgene.
6. A process according to any one of Claims 1 to 5, wherein the solvent capable of forming a Vilsmeier reagent is selected from dimethylformamide, n-alkyl-pyrrolidinone and n-formylpiperidine.
7. A process according to Claim 6, wherein the solvent capable of forming a Vilsmeier reagent is dimethylformamide.
8. A process according to Claim 1, wherein $C_8$-$C_{24}$ alkylbenzenesulfonic acid is reacted in a suitable water-immiscible, nonreactive solvent with thionyl chloride, phosphoryl chloride or phosgene in the presence of a solvent capable of forming a Vilsmeier reagent at about 70 °C.
9. A process according to any one of Claims 1 to 8, wherein the phase transfer catalyst useful for anion transfer is selected from quaternary ammonium or phosphonium salts, macrocyclic or macrobicyclic « crown » ethers, triazine ethers or N-alkyl phosphoramides.
10. A process according to Claim 9, wherein the phase transfer catalyst useful for anion transfer is a quaternary salt.
11. A process according to Claim 10, wherein the quaternary salt is tri-n-$C_8$-$C_{10}$ alkylmethyl ammonium chloride.

**Patentansprüche**

1. Verfahren zur Herstellung von $C_8$-$C_{24}$-Alkylbenzolsulfonylaziden, umfassend :
a) Umsetzung von $C_8$-$C_{24}$-Alkylbenzolsulfonsäuren in einem geeigneten, mit Wasser nicht-mischbaren, nicht-reaktiven Lösungsmittel mit einem Überschuss eines reaktive Chlorierungsmittels, in Gegenwart eines zur Bildung eines Vilsmeier-Reagenz fähigen Lösungsmittels, zwischen 40° und 70 °C unter Bildung von $C_8$-$C_{24}$-Alkylbenzolsulfonylchloriden und Entfernung von verbleibenden Säuren durch Waschen mit Natriumbicarbonat und anschliessend
b) Umsetzung der $C_8$-$C_{24}$-Alkylbenzolsulfonylchloride im genannten, mit Wasser nicht-mischbaren, nicht-reaktiven Lösungsmittel mit wässerigem Natriumazid in Gegenwart eines zur Anionenübertragung geeigneten Phasenübertragungskatalysators.
2. Verfahren nach Anspruch 1, wobei es sich bei den $C_8$-$C_{24}$-Alkylbenzolsulfonylaziden um 4-Dodecylbenzolsulfonazide handelt.
3. Verfahren nach Anspruch 1 oder 2, wobei das geeignete, mit Wasser nicht-mischbare, nichtreaktive Lösungsmittel ausgewählt ist aus Hexan, Toluol und Methylenchlorid.
4. Verfahren nach Anspruch 3, wobei es sich bei dem geeigneten, mit Wasser nicht-mischbaren, nicht-reaktiven Lösungsmittel um Hexan handelt.
5. Verfahren nach einem der Ansprüche 1 bis 4, wobei die $C_8$-$C_{24}$-Alkylbenzolsulfonsäuren mit Thionylchlorid, Phosphorylchlorid oder Phosgen umgesetzt werden.
6. Verfahren nach einem der Ansprüche 1 bis 5, wobei das zur Bildung eines Vilsmeier-Reagenz geeignete Lösungsmittel ausgewählt ist aus Dimethylformamid, n-Alkylpyrrolidinon und n-Formylpiperidin.
7. Verfahren nach Anspruch 6, wobei es sich bei dem zur Bildung eines Vilsmeier-Reagenz fähigen Lösungsmittel um Dimethylformamid handelt.
8. Verfahren nach Anspruch 1, wobei die $C_8$-$C_{24}$-Alkylbenzolsulfonsäure in einem geeigneten, mit Wasser nicht-mischbaren, nicht-reaktiven Lösungsmittel mit Thionylchlorid, Phosphorylchlorid oder

Phosgen in Gegenwart eines zur Bildung eines Vilsmeier-Reagenz fähigen Lösungsmittels bei etwa 70 °C umgesetzt wird.

9. Verfahren nach einem der Ansprüche 1 bis 8, wobei der zur Anionenübertragung geeignete Phasenübertragungskatalysator ausgewählt ist aus quaternären Ammonium- oder Phosphoniumsalzen, macrocyclischen oder macrobicyclischen Kronenäthern, Triazinäthern oder n-Alkylphosphorsäureamiden.

10. Verfahren nach Anspruch 9, wobei der für die Anionenübertragung geeignete Phasenübertragungskatalysator ein quaternäres Salz ist.

11. Verfahren nach Anspruch 10, wobei das quaternäre Salz Tri-n-$C_8$-$C_{10}$-Alkyl-methylammoniumchlorid ist.

**Revendications**

1. Procédé de préparation d'azotures d'alkyl(en $C_8$-$C_{24}$) benzène-sulfonyle comprenant les opérations qui consistent :

a) à faire réagir des acides alkyl(en $C_8$-$C_{24}$) benzène-sulfoniques, dans un solvant convenable, non miscible à l'eau et non réactif, avec un excès d'un agent de chloration réactif, en présence d'un solvant capable de former un réactif de Vilsmeier, à une température comprise entre 40° et 70 °C, pour former des chlorures d'alkyl(en $C_8$-$C_{24}$) benzène-sulfonyle et à éliminer tous les acides restant éventuellement avec un lavage au bicarbonate de sodium, puis

b) à faire réagir lesdits chlorures d'alkyl(en $C_8$-$C_{24}$) benzène-sulfonyle dans ledit solvant non miscible à l'eau et non réactif avec de l'azoture de sodium aqueux, en présence d'un catalyseur de transfert de phase utile pour le transfert d'anions.

2. Procédé selon la revendication 1, dans lequel les azotures d'alkyl(en $C_8$-$C_{24}$) benzène-sulfonyle sont des azotures de 4-dodécylbenzène-sulfonyle.

3. Procédé selon la revendication 1 ou 2, dans lequel le solvant convenable, non miscible à l'eau et non réactif, est choisi parmi l'hexane, le toluène et le chlorure de méthylène.

4. Procédé selon la revendication 3, dans lequel le solvant convenable, non miscible à l'eau et non réactif, est l'hexane.

5. Procédé selon l'une quelconque des revendications 1 à 4, dans lequel on fait réagir les acides alkyl(en $C_8$-$C_{24}$) benzène-sulfoniques avec du chlorure de thionyle, du chlorure de phosphoryle ou du phosgène.

6. Procédé selon l'une quelconque des revendications 1 à 5, dans lequel le solvant capable de former un réactif de Vilsmeier est choisi parmi le diméthylformamide, une N-alkyl-pyrrolidinone et la N-formylpipéridine.

7. Procédé selon la revendication 6, dans lequel le solvant capable de former un réactif de Vilsmeier est le diméthylformamide.

8. Procédé selon la revendication 1, dans lequel on fait réagir un acide alkyl(en $C_8$-$C_{24}$) benzène-sulfonique, dans un solvant convenable, non miscible à l'eau et non réactif, avec du chlorure de thionyle, du chlorure de phosphoryle ou du phosgène, en présence d'un solvant capable de former un réactif de Vilsmeier, à environ 70 °C.

9. Procédé selon l'une quelconque des revendications 1 à 8, dans lequel le catalyseur de transfert de phase utile pour le transfert d'anions est choisi parmi les sels d'ammonium ou de phosphonium quaternaire, les éthers « couronne » macrocycliques ou macrobicycliques, les éthers de triazine ou les N-alkylphosphoramides.

10. Procédé selon la revendication 9, dans lequel le catalyseur de transfert de phase utile pour le transfert d'anions est un sel quaternaire.

11. Procédé selon la revendication 10, dans lequel le sel quaternaire est un chlorure de tri-n-(alkyl en $C_8$-$C_{10}$) méthylammonium.